# EUROPEAN PATENT SPECIFICATION

(11) **EP 1 648 523 B1**
(45) Date of publication and mention of the grant of the patent: **03.09.2008**
(21) Application number: 04743563.1
(22) Date of filing: 26.07.2004
(51) Int. Cl.: A61L 2/16, A61L 26/00, A61L 2/26, A01N 25/34, C11D 17/04, D21H 21/36, A61L 2/00, A61L 15/00, A61F 13/00, A61F 15/00, A61L 2/23

(54) **STERILANT SYSTEM**
STERILISIERSYSTEM
SYSTEME STERILISANT

(30) Priority: 29.07.2003 GB 0317750; 26.09.2003 GB 0322589; 07.05.2004 GB 0410203
(43) Date of publication of application: 26.04.2006
(73) Proprietor: Tristel PLC, Fordham Road, Snailwell Newmarket CB8 7NY (GB)
(72) Inventor: Green, Bruce Philip, Northampton, NN6 7BP (GB)
(74) Representative: Gemmell, Peter Alan
(86) International application number: PCT/GB2004/003234
(87) International publication number: WO 2005/011756

(56) References cited:
- WO-A-96/10916
- US-A- 4 978 530
- US-A- 5 213 884
- US-A- 5 227 168
- US-A- 5 783 146

## Description

The present invention relates to a two-part sterilant system, notably to a system for producing chlorine dioxide (ClO₂). The invention is particularly for use in sterilising medical supplies and equipment, but it is not limited to these uses.

### BACKGROUND TO THE INVENTION

Two-part sterilising solutions are used in applications where the active sterilising ingredient is unstable over time. The solution is therefore prepared *in situ* shortly before it is to be used. A particularly important sterilising agent is chlorine dioxide, which may be formed from mixtures of various reagents including: chlorite and acid; chlorate, peroxide and acid; and chlorite, hypochlorite, and a suitable buffer. Chlorine dioxide has excellent sterilising and bactericidal properties, and oral ingestion in man and animals has been shown to be relatively safe.

The cleaning of endoscopes and other medical equipment with suitable chlorine dioxide solutions is known from earlier patents in the name of the present inventor, for example, European Patent Number 0 785 719 and United States Patent Numbers 5,696,046 and 6,007,772.

It is not always convenient to mix up batches of solutions for use in sterilising equipment. For wiping down (rather than thoroughly cleaning inside and out) of endoscopes and probes, wipes of alcohol, general-purpose detergent, or soapy water are generally used, but these are not as effective as chlorine dioxide. It is desirable to be able readily to make up small quantities of two-component sterilising agents when desired and to be able to make such agents up in a form in which they may be readily handled for a particular application.

### SUMMARY OF THE INVENTION

According to a first aspect of the present invention there is provided a two-part sterilant system according to claim 1.

It is particularly preferred that the first part is dispensed as a foam to facilitate its coverage of a desired area of the fabric member. Optionally, the dispenser may have a relatively large dispensing head, for supplying the foam over all or a substantial part of a surface of the fabric member. For example, the dispensing head may take the form of a rose or sprinkler with a multitude of small orifices to spread the foam over the fabric member.

The pump dispenser is preferably a trigger-operated dispenser, both for convenience and to facilitate the dispensing of metered quantities. However, other pump dispensers could be used, for example, a squeeze bottle with a suitable spray or foam nozzle. The invention will, for convenience, be described hereinafter with reference to the use of a trigger-operated dispenser, but it is to be understood that it is not limited to this embodiment.

By putting up the first part in a trigger-operated dispenser, small quantities may be readily dispensed without risk of spillage. Preferably the dispenser comprises a sprayer apparatus that provides the first part as a foam so that it is at least partly form-retaining and can be readily seen and manipulated. We have also found that providing the first part in a foam may have the beneficial effect of reducing the odour of chlorine dioxide when the wipe is activated. The invention will for convenience be described with reference to this preferred embodiment, but it will be understood that the invention is not limited to this embodiment.

The trigger sprayer may include a mixing chamber to facilitate mixing of the first part with air, for example as described in United States patent number 5,337,929.

The fabric members may be formed from any suitable fabrics, either woven or non-woven. They may be of natural or man-made fibres, for example polyester, cotton, cellulose or mixtures thereof. Other suitable fabrics will be well known to those skilled in the textile or fabric arts.

The fabric member may comprise a fabric wipe or cloth, or a gauze, pad, or other wound dressing material. Once prepared, the fabric member will have biocidal properties and may be used to dress wounds, ulcers, or the like while promoting a sterile local environment around the wound. For convenience, the invention will be described hereinafter with reference to the use of a fabric wipe, but it is to be understood that the invention is not limited to this embodiment.

By providing the second part absorbed in a fabric wipe, a sterilising wipe may readily be prepared by applying the first part to the wipe. The user may fold the wipe or rub two halves together to facilitate mixing. The wipes are particularly useful for cleaning, disinfecting, and sterilising surfaces and equipment, notably in a medical environment.

The first part may include a coloured component so that a visual indication of the coverage of the wipe with the first part can be made.

In a preferred embodiment, at least one of the first and second parts is provided with an indicator reagent that changes colour to show that sufficient mixing has taken place. Where the first part and the second part are of different pH, the indicator may be a pH-sensitive indicator. Suitable indicators are well known to those skilled in the art, non-limiting examples including: phenol red, litmus, thymol blue, pentamethoxy red, tropeolin OO, 2,4-dinitrophenol, methyl yellow, methyl orange, bromophenol blue, tetrabromophenol blue, alizarin sodium sulphonate, α-naphthyl red, p-ethoxychrysoidine, bromocresol green, methyl red, bromocresol purple, chlorophenyl red, bromothymol blue, p-nitrophenol, azolitmin, neutral red, rosalic acid, cresol red, α-naphtholphthalein, tropeolin OOO, phenolphthalein, α-naphtholbenzein, thymolphthalein, nile blue, alizarin yellow, diazo violet, tropeolin O, nitramine, Poirrer's blue, trinitrobenzoic acid, and mixtures thereof. It is preferred that the indicator is selected so that both parts are separately colourless and the colour develops when the two parts are mixed.

Alternatively, or additionally, one or more fluorescent additives may be included so that the mixture fluoresces to indicate mixing. Non-limiting examples of suitable fluorescing agents include: 4-methylumbelliferone, 3,6-dihydroxanthone, quinine, thioflavin, 1-napthol, harmine, coumarin, acridine orange, cotarmine, and mixtures thereof.

The indicator (colour change or fluorescent) may be included in either part. Preferred proportions by weight are about 0.1 to 10%, notably about 0.5 to 2%.

The carrier mediums may be fluids such as liquids or sols, or they may be more form-retaining or viscous compositions such as gels or pastes. It is preferred that at least one reagent is present in an aqueous fluid, although other additives may of course be present. Preferably both reagents are put up in aqueous fluids.

The trigger-operated dispenser may be a conventional foamer, or other manual pump in which the contents are expelled manually by operation of the trigger by the user. Alternatively, the dispenser may contain a propellant to dispense the contents when operation of the trigger opens a valve, as is well known in applications such as shaving foam canisters and the like. Suitable dispensers will be well known to those skilled in the art.

The preferred sterilising agent is chlorine dioxide, which may be formed from suitable known reagents. In a preferred embodiment one reagent is a chlorite (notably sodium chlorite) and the other is an acid, preferably with a buffer. Suitable acids include lactic acid, citric acid, boric acid, phosphoric acid, acetic acid, sorbic acid, ascorbic acid, hydrochloric acid or mixtures thereof. In a preferred embodiment a mixture of acids is used, notably a mixture of citric, sorbic and boric acids.

A particularly preferred system is as described in EP 0 785 719, with the corrosion inhibitors optionally not included, and with other additives as desired for particular applications. In addition to suitable indicators, optional additives include foam-promoting agents or stabilisers, humectants, essential oils and fragrances. Other sterilising agents may also be employed; for example chlorine or oxygen. Chlorine may be produced by reaction between a hypochlorite such as sodium hypochlorite, and a suitable acid or buffer. Oxygen may be produced by reaction between a peroxide and a catalyst such as catalase, optionally in the presence of a buffer. For convenience hereinafter, the invention will be described with reference to chlorine dioxide as the sterilising agent.

Suitable foam promoters will be well known to those skilled in the art. Non-limiting examples include: sodium laureth sulphate, ammonium lauryl sulphate, cocamide DEA, cocamidopropyl betaine, sodium lauryl sarcosinate, cocamidopropylamine oxide, monoethanolamine lauryl sulphate, cocamidopropyl hydroxysultaine, cocoyl sarcosinate. Anionic, cationic, non-ionic and amphoteric surfactants may be employed depending on the chemistry of the reagents. The foam promoters are selected to provide a stable foam structure. The foam promoter may comprise from about 0.1 to 50% by weight of the first part, notably from about 1 to 10%, preferably from about 3 to 6%.

Suitable foam stabilisers well known to those skilled in the art may also be used, in proportions similar to those for the foam-promoters. Non-limiting examples include: alkanolamides, for example monoethanolamides and diethanolamides, amine oxides, betaines, protein hydrolysates and cellulose derivatives such as carboxymethylcellulose.

In a preferred embodiment, a humectant is included in at least one of the first and second parts. Humectants serve to reduce the rate of evaporation of components and improve product feel if direct skin contact is involved. We have found that the use of a humectant reduces the volatility of chlorine dioxide, which reduces the odour of chlorine dioxide and prolongs the life of the activated mixture. Non-limiting examples of suitable humectants include sodium lactate and polyols, for example glycerine, sorbitol, propylene glycol, diethylene glycol and ethylene glycol. The humectant may be present in any desired amount, particularly from about 0.1 to 50% by weight, notably from about 0.5 to 10%, preferably from about 1 to 3%.

Where one of the reagents is basic or oxidising, for example sodium chlorite, it is particularly preferred that this reagent is provided in the trigger dispenser rather than in the wipe, because such reagents may react with the fabric over time. Preferably the optional humectant is included in the first part, with the sodium chlorite or other first reagent.

The first and/or second part may further include a biocide to ensure that, in the event of poor mixing of the parts, a biocidal effect is still present. The first and/or second part may also include a preservative.

Equal weights of the first part and the second part may provide, when mixed, a sterilising composition having a pH of from 1.0 to 10.5, but it is preferred that the composition has a pH of from 4.5 to 6.5 as this may result in a more stable compound.

A plurality of fabric members may be provided in a single resealable container, for example a canister with a lid, or a resealable sachet. In a preferred embodiment, each fabric member is provided in its own sachet which may be factory-sealed and disposed of after use. In a particularly preferred embodiment, each sealed sachet contains a single fabric wipe.

Other aspects and benefits of the invention will appear in the following specification, drawings and claims.

### BRIEF DESCRIPTION OF THE DRAWINGS

The invention will now be further described, by way of example, with reference to the following drawings in which:
Figure 1 shows a trigger sprayer for use in a sterilant system in accordance with an embodiment of the present invention;
Figures 2 and 4 show an canister of sterilising wipes, respectively open and closed, for use in a sterilant system in accordance with an embodiment of the present invention;
Figure 3 shows an alternative trigger sprayer for use in a sterilant system in accordance with an alternative embodiment of the invention; and
Figure 5 illustrates a sterilant system in accordance with a further embodiment of the invention.

### DETAILED DESCRIPTION

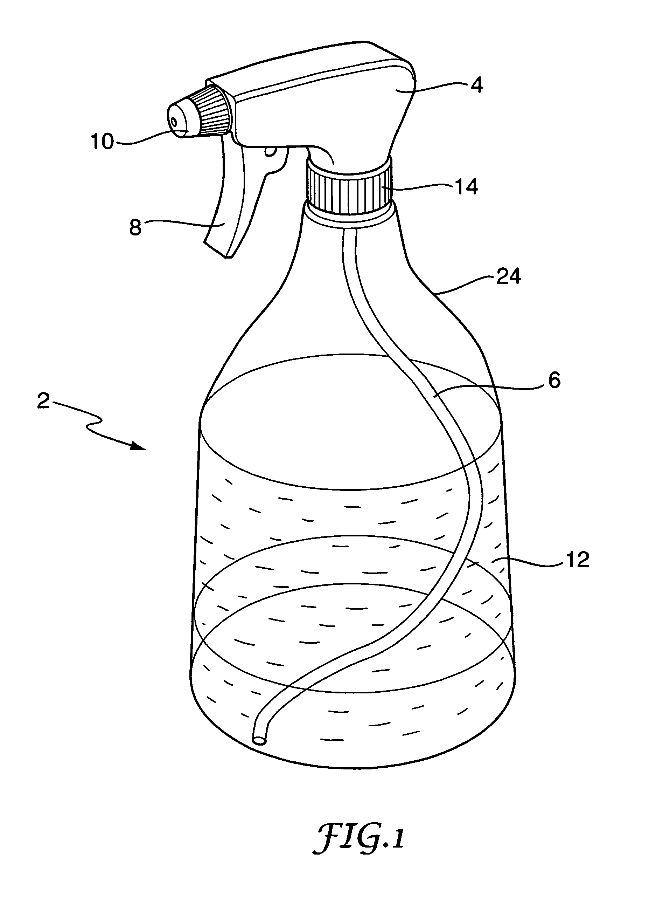

The trigger sprayer shown in Figure 1 is of a construction well known *per se.* The sprayer comprises a body 2 connected to a sprayer head 4 by an internally screw-threaded connector ring 14. A spray nozzle 10 in the head is connected to an aqueous liquid 12 by means of a dip tube 6. A user dispenses the liquid 12 through the nozzle 10 by operation of a trigger 8.

In the present example, the liquid 12 (first part) comprises 0.75% of a first reagent (sodium chlorite), 3.0% foam promoter (Cocamidopropyl Betaine). The remainder is deionised water. In this specification, all parts are by weight unless otherwise indicated. Operation of the trigger 8 dispenses the first part 12 as a foam.

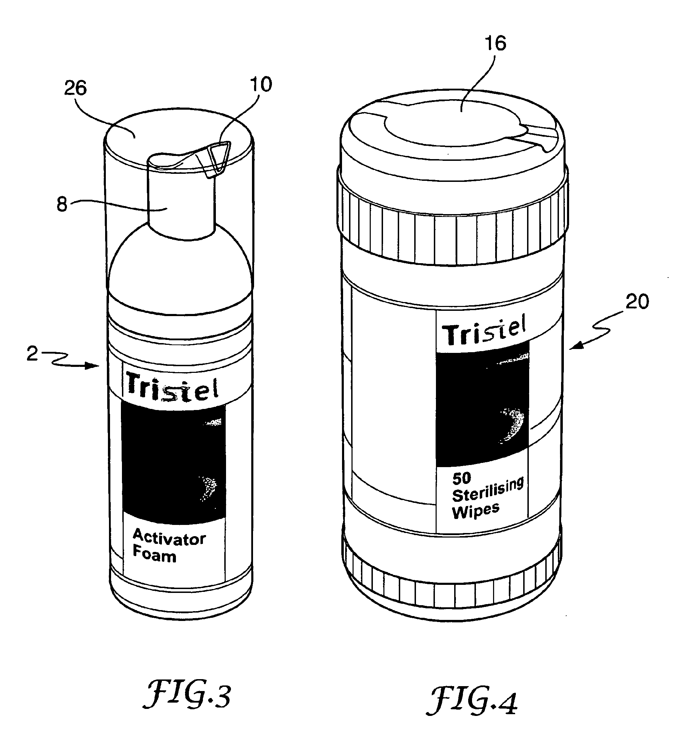

An alternative design of pump dispenser 2 is illustrated in Figure 3. The trigger 8 is formed integrally with the nozzle 10. Depressing the trigger 8 dispenses a portion of the fluid contents as a foam (referred to as the 'Activator Foam' because it activates the sterilising powers of a sterilising wipe). A protective cap 26 is provided to cover the nozzle 10 and trigger 8 when not in use.

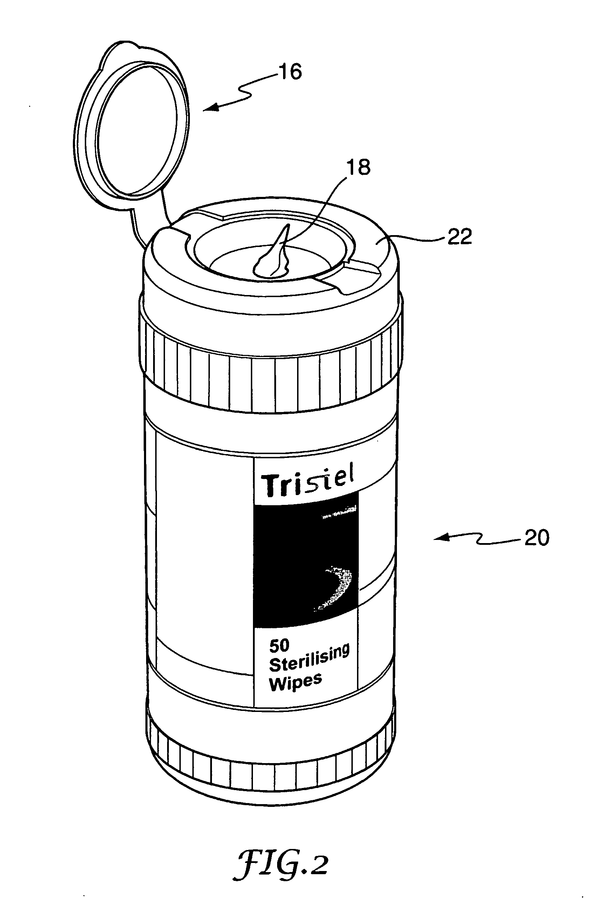

Turning now to Figures 2 and 4, a sealable container 20 is also of a construction well known *per se.* The container 20 is a hollow cylinder fitted with a cap 22. The container 20 contains a roll of interleaved fabric sheets 18. In this example, the fabric sheets 18 are to be used as sterilising wipes, but it will be understood that the sheets 18 could also be used for other applications such as biocidal wound-dressings.

The cap 22 has a central opening through which a tip of the central wipe 18 is disposed. By pulling the central wipe 18, a user may remove this wipe from the container, leaving the next wipe in its place. A stopper 16 is provided on the cap 22 for releasably sealing the container 20.

In this example, the wipes 18 are impregnated with an aqueous acid solution (second part). In this example, the acid solution comprises 0.5% citric acid, 0.05% sorbic acid, 0.05% boric acid. The solution also comprises 0.35% of a buffer (trisodium phosphate). The solution also comprises 0.25% Trisodium Citrate, 1.0% glycerine, 0.1% Benzotriazole, 0.1% Sodium Molybdate and 0.3% Sodium Nitrate. The remainder is deionised water.

The pump dispenser 2 and container 20 together comprise the sterilant system. To prepare a sterilising wipe, a user removes an impregnated wipe 18 from the container 20, and applies a portion of foam from the sprayer 2 to the wipe 18. To facilitate mixing of the reagents in the foam and the wipe, the user may fold the wipe in half and crush or rub the folded wipe before opening it out. Preferably, one of the components is provided with a pH-sensitive indicator which changes colour or becomes coloured when adequate mixing has occurred, thereby indicating that sufficient ClO₂ has been generated in the wipe.

Once the sterilising wipe has been prepared, it may be used for a number of applications, including wiping surfaces and sterilising medical equipment such as endoscopes.

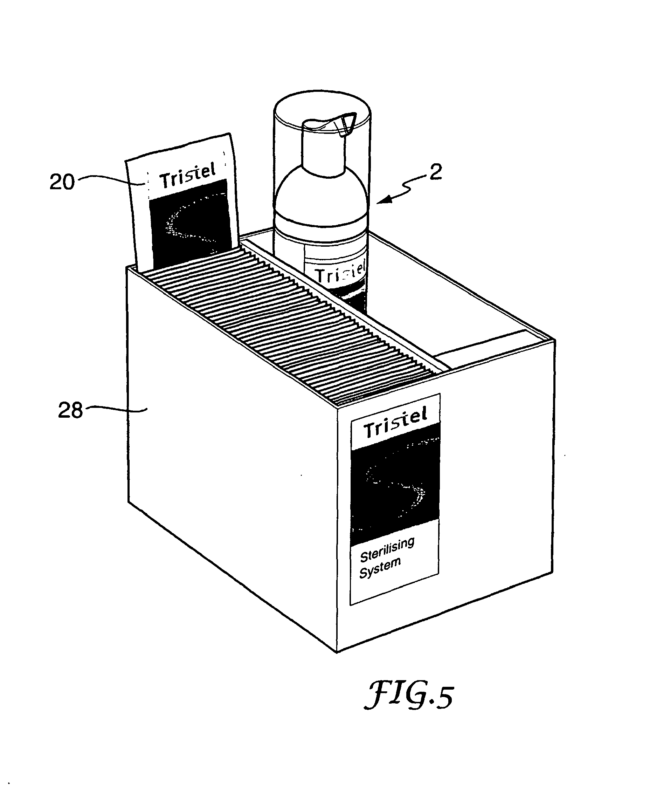

In the preferred embodiment illustrated in Figure 5, each sterilising wipe 18 is provided in its own sealed container 20, in this example a sachet. The sterilant system comprises a box 28 of sterilising wipes 18 in individual sachets 20. Each sachet 20 may be factory sealed and may be disposed of after the wipe has been removed. The foam pump dispenser 2 is also provided in the box 28 of sterilising wipe sachets 20.

### EXPERIMENTAL RESULTS

### Experiment 1

Sterilant wipes in accordance with one embodiment of the invention were tested and compared with conventional wipes saturated with isopropanol (IPA), a general-purpose detergent, and sterile deionised water.

The test method to evaluate effectiveness of the wipes in killing/removing test organisms dried onto test surfaces, involved the following steps.
1. Mark out a six inch (30.5 cm) square test area on the test surface.
2. Inoculate the test surface with 0.5 ml of test organism suspension.
3. Spread the inoculum over the test area using a plastic spreader.
4. Allow the inoculum to dry (about 30 minutes).
5. Don a pair of disposable plastic gloves.
6. Prepare a ClO₂ wipe in accordance with the invention, using a prescribed mixing time.
7. Wipe the test area for the prescribed wiping time.
8. Place the wipe in 10 ml of universal neutraliser in a Universal bottle (Test Suspension A). Vortex stir to release organisms.
9. Wipe the entire test area with a cotton-tipped swab (thoroughly/10 times).
10. Dip the swab into 10 ml of universal neutraliser in a Universal bottle after each sampling of the test area and rotate the swab against the inner wall of the bottle to release organisms (Test Suspension B).
11. Prepare 5 serial deci-dilutions of Test Suspension A and Test Suspension B in diluent.
12. Inoculate 0.5 ml of each dilution onto a culture plate and spread using a plastic spreader. Incubate the plates and do a viable count.
13. Calculate log₁₀ reductions achieved from the difference in the initial inoculum and the number of test organisms recovered after disinfection with a ClO₂ wipe.

Test variables were as follows.

### Test Surface

A flat stainless steel instrument tray.

### Test Organism

Spores of Bacillus subtilis var.niger NCTC 10073 freshly prepared by the method of Beeby & Whitehouse.

### Inoculum

The test surface was inoculated with 1 x 10⁸ spores.

### Suspending Fluid

Sterile deionised water.

### Disinfectant Concentrations

1. 200 ppm ClO₂ (notional)
2. 300 ppm ClO₂ (notional).

### Mixing Times

15 + 30 seconds.

### Wiping Times

15 + 30 + 60 seconds.

### Controls

1. 1% Hospec general purpose neutral liquid detergent (Young's Detergents)/Kimcare Medical Wipes (Kimberly-Clark).
2. Sterets Alcowipe: 70% IPA (Seton Prebbles Ltd).
3. Sterile deionised water: Kimcare Medical Wipes (Kimberly-Clark).

Results are given in Table 1.

**TABLE 1**

| Exp. No. | Disinfectant/Detergent | Mixing time (seconds) | Wiping time (seconds) | VC Surface | VC Wipe |
|---|---|---|---|---|---|
| 1 | 200 ppm ClO₂ | 15 | 15 | 177 | 143 |
| 2 | 200 ppm ClO₂ | 15 | 30 | 36 | 14 |
| 3 | 200 ppm ClO₂ | 15 | 60 | 10 | 8 |
| 4 | 200 ppm ClO₂ | 30 | 15 | 800 | 300 |
| 5 | 200 ppm ClO₂ | 30 | 30 | 240 | 27 |
| 6 | 200 ppm ClO₂ | 30 | 60 | 29 | 26 |
| 7 | 300 ppm ClO₂ | 15 | 15 | 1240 | 330 |
| 8 | 300 ppm ClO₂ | 15 | 30 | 530 | 250 |
| 9 | 300 ppm ClO₂ | 15 | 60 | 160 | 140 |
| 10 | 300 ppm ClO₂ | 30 | 15 | 1450 | 900 |
| 11 | 300 ppm ClO₂ | 30 | 30 | 30 | 70 |
| 12 | 300 ppm ClO₂ | 30 | 60 | 20 | 10 |
| 13 | 1% Hospec | | 60 | 7.3x10⁴ | 4.3x10⁵ |
| 14 | 70% IPA | | 60 | 1.9x10⁴ | 3.7x10⁴ |
| 15 | Deionised H₂O | | 60 | 2.0x10⁵ | 3.0x10⁵ |

| | | | | | |
|---|---|---|---|---|---|
| VC = Viable Count | | | | | |

### Interpretation of Results

1. Washing/wiping with water, neutral detergent (1% Hospec), or alcohol (70% IPA) were ineffective
2. For the notional 200 ppm ClO₂ wipes the best results were obtained with a mixing time of 15 seconds and a wiping time of 60 seconds.
3. For the notional 300 ppm ClO₂ wipes the best results were obtained with a mixing time of 30 seconds and a wiping time of 60 seconds.
4. Results for 200 ppm ClO₂ (notional) were surprisingly better than results for 300 ppm (notional), except for mixing times of 30 seconds combined with wiping times of at least 30 seconds.
5. A wiping time of 60 seconds achieved better results than a wiping time of 30 seconds, which in turn achieved better results than a wiping time of 15 seconds.
6. Both ClO₂ concentrations achieved good results after a wiping time of 60 seconds. The test surface was inoculated with 1 x 10⁸ spores. After using the ClO₂ wipes, surface counts were reduced to 10 and 29 (200 ppm ClO₂) and to 160 and 20 (300 ppm ClO₂).
7. A wipe containing 200 or 300 ppm may be useful, as may mixing times of 15 or 30 seconds (or, clearly, any intermediate times). However, it is preferred that wiping times longer than 15 seconds are employed.

These results were obtained using bacterial spores. It is to be expected that a vegetative bacterium such as MRSA will be much more sensitive, so that lower ClO₂ concentrations and/or shorter mixing or wiping times may be effective against such bacteria.

Further experiments (2-4) were carried out using 41 gsm spunlace sheets comprised of 50.5% wood pulp and 49.5% PET. The sheets' dimensions were 160 mm x 180 mm x 0.36 mm. In each experiment the wipes each contained 3 ml of Solution A (formulated as set forth below), made by treating a canister of 50 wipes with 150 ml of Solution A. Each wipe was activated with 1.5 ml of Solution B (formulated as set forth below) from a foam dispenser.

### Solution A (Wipe)

### Formulation:

| Ingredients | | Actual % w/w | Tolerance |
|---|---|---|---|
| 1 | Citric acid | 0.50% | +/- |
| | C.A.S. 77-92-9 | | 0.60-0.40% |
| 2 | Sorbic acid | 0.005% | +/- |
| | C.A.S. | | 0.006- |
| | | | 0.004% |
| 3 | Boric acid | 0.005% | +/- |
| | C.A.S. 10043-35-3 | | 0.006- |
| | | | 0.004% |
| 4 | Trisodium citrate | 0.25% | +/- |
| | C.A.S. 68-04-02 | | 0.30-0.20% |
| 5 | Trisodium phosphate | 0.35% | +/- |
| | C.A.S. 10101-89-0 | | 0.45-0.25% |
| 6 | Glycerin | 1.00% | +/- |
| | C.A.S. 56-81-5 | | 1.10-0.90% |
| 7 | Benzotriazole | 0.10% | +/- |
| | C.A.S. 95-14-7 | | 0.15-0.05% |
| 8 | Sodium molybdate | 0.10% | +/- |
| | C.A.S. 10102-40-6 | | 0.15-0.05% |
| 9 | Sodium nitrate | 0.20% | +/- |
| | C.A.S. 7631-99-4 | | 0.25-0.15% |
| 10 | Preservative (Paramotol) | 0.15% | +/- |
| | C.A.S. | | 0.20-0.10% |
| 11 | Deionised water | Balance | Balance |
| | C.A.S. 7732-18-5 | | |

### Solution B (Foam)

### Formulation:

| Ingredients | | Actual % w/w | Tolerance |
|---|---|---|---|
| 1 | Sodium chlorite (25% solution) | 0.75% | +/- |
| | | | 0.85- |
| | | | 0.65% |
| 2 | Cocamidopropyl betaine | 3.00% | +/- |
| | | | 3.10- |
| | | | 2.90% |
| 3 | Indicator/colour solution | 0.60% | +/- |
| | (Indicator is cosmetic yellow, | | 0.07- |
| | No. 5, cl 19140 at 1% solution | | 0.50% |
| | 0.6%) | | |
| 4 | Preservative | 0.15% | +/- |
| | (Euxyl K 100) | | 0.20- |
| | | | 0.10% |
| 5 | Deionised Water (Purified) | 95.50% | +/- |
| | C.A.S. 7732-18-5 | | Balance |

### Experiment 2

A study was carried out to compare the effectiveness of (a) ClO₂ wipes in accordance with the invention (b) a 70% IPA wipe (c) a neutral detergent wipe and (d) a water wipe in removing and/or killing (1) B. subtilis spores, and (2) P. aeruginosa cells dried onto the insertion tube of a flexible endoscope.

Wipes were prepared fresh as required by squirting foam onto a wipe and then scrunching the wipe with the fingers to mix the reagents to form ClO₂.

### EXPERIMENT 2

### Test organisms

### B. subtilis NCTC 10073 spores

A suspension containing approximately 10⁸ spores/ ml was prepared by the method of Beeby & Whitehouse. A 1 in 10 dilution in sterile distilled water was prepared to produce a suspension containing approximately 10⁷ spores/ ml.

### P. aeruginosa NCTC 6749

A culture containing approximately 10⁸ cells/ ml was prepared by inoculating a tube of nutrient broth and incubating for 18 h at 37° C.

### Insertion tube used in Experiment 2

The insertion tube was 1 metre long, in good condition, with clear markings. The test site used was the 10 cm section between the 30 and 40 markings.

### Test Method

1. Immerse a cotton-tipped swab into a suspension of spores or vegetative cells.
2. Inoculate entire surface area of test site with the suspension. Repeat several times. Regarding *B. subtilis* spores, assume that (1) the volume of inoculum = 0.1 ml, and (2) the mortality rate on drying out is zero. Hence the viable count of the inoculum = approximately 10⁶ spores. Regarding *P*. *aeruginosa* cells, assume that (1) the volume of inoculum = 0.1 ml, and (2) the mortality rate on drying out is 1 log. Hence the viable count of the inoculum = approximately 10⁶ cells.
3. Place inoculated insertion tube across the top of an empty discard jar with the 10 cm test site resting over the centre of the jar. Allow inoculum to dry out (approximately 30 minutes).
4. Don pair of disposable plastic gloves.
5. Prepare a Wipe: ClO₂ (scrunch time = 15 sec), IPA, Hospec or water.
6. Wipe test site for the prescribed wipe time (30 sec) as follows: Wrap wipe loosely around the insertion tube and then wipe up and down the test site repeatedly.
7. Place the wipe in 20 ml of universal neutraliser in a Universal bottle. Vortex stir to release recovered spores/cells (Test Suspension A).
8. Swab entire test site with a cotton-tipped swab. Dip swab into 10 ml of universal neutraliser in a Universal bottle and rotate swab against the inner wall of the bottle to release recovered spores/ cells. Repeat 10 times then break off cotton-tip of swab and leave in the neutraliser. Vortex stir to release recovered spores/ cells (Test Suspension B).
9. Prepare 5 serial deci-dilutions of Test Suspension A and Test Suspension B in diluent.
10. Inoculate 0.5 ml of each dilution onto a culture plate and spread using a plastic spreader. Incubate plates. Viable count.
11. Calculate log₁₀ reductions achieved from the difference in the number of spores or cells inoculated onto the test site (approximately 10⁶) and the number recovered after cleaning and/or disinfection.

### Wipes used in Experiment 2

1. ClO₂ Wipe (scrunch time = 15 seconds).
2. 70% IPA wipe: Azowipe (Vernon Carus).
3. Hospec wipe: Kimberley Clark Medical Wipe immersed in 1% Hospec and then squeezed to remove excess solution.
4. Water wipe: Kimberley Clark Medical Wipe immersed in sterile water and then squeezed to remove excess water.

### EXPERIMENT 2 - RESULTS

**Table 2**

| Exp | Test organism | Disinfectant/detergent | Scrunch time (sec) | Wipe time (sec) | Viable Count (0.5 ml) | |
|---|---|---|---|---|---|---|
| | | | | | Surface | Wipe |
| 1 | *B. subtilis* | ClO₂ | 15 | 30 | 0 | 0 |
| 2 | | ClO₂ (repeat) | 15 | 30 | 0 | 0 |
| 3 | | 70% IPA | | 30 | 5.0 x 10² | 2.7 x 10³ |
| 4 | | 1% Hospec | | 30 | 1.5 x 10² | 2.6 x 10³ |
| 5 | | Water | | 30 | 3.0 x 10¹ | 2.5 x 10³ |
| 6 | *P. aeruginosa* | ClO₂ | 15 | 30 | 0 | 0 |
| 7 | | ClO₂ (repeat) | 15 | 30 | 0 | 0 |
| 8 | | 70% IPA | | 30 | 2 | 0 |
| 9 | | 1% Hospec | | 30 | 6.2 x 10³ | 8.0 x 10⁴ |
| 10 | | Water | | 30 | 2.5 x 10⁴ | 1.5 x 10⁵ |

**Table 3**

| Exp | Test organism | Disinfectant/ detergent | Total spores/ cells recovered | |
|---|---|---|---|---|
| | | | Surface¹ | Wipe² |
| 1 | *B. subtilis* | ClO₂ | 0 | 0 |
| 2 | | ClO₂ (repeat) | 0 | 0 |
| 3 | | 70% IPA | 1.0 x 10⁴ | 1.0 x 10⁵ |
| 4 | | 1% Hospec | 3.0 x 10³ | 1.0 x 10⁵ |
| 5 | | Water | 6.0 x 10² | 1.0 x 10⁵ |
| 6 | *P. aeruginosa* | ClO₂ | 0 | 0 |
| 7 | | ClO₂ (repeat) | 0 | 0 |
| 8 | | 70% IPA | 4.0 x 10¹ | 0 |
| 9 | | 1% Hospec | 1.2 x 10⁵ | 3.2 x 10⁶ |
| 10 | | Water | 5.0 x 10⁵ | 6.0 x 10⁶ |

| | | | | |
|---|---|---|---|---|
| ¹ Viable count in Table 1 x 20 (0.5 ml of 10 ml neutraliser plated out). ² Viable count in Table 1 x 40 (0.5 ml of 20 ml neutraliser plated out). | | | | |

### EXPERIMENT 2 - CONCLUSIONS

1. ClO₂ wipes were completely effective against both *B. subtilis* spores and *P. aeruginosa* cells. No spores or cells were recovered in duplicate experiments.
2. IPA wipes exhibited good activity against *P. aeruginosa* cells but did not eliminate all of the test cells - 40 viable cells were recovered from the test site on the insertion tube.
3. IPA wipes were ineffective against *B. subtilis* spores. IPA proved less effective than 1% Hospec or water which may be attributable to the coagulant properties of alcohol (fixing spores on the test site).
4. Wipes saturated with 1% Hospec were ineffective against either *B. subtilis* spores or *P. aeruginosa* cells.
5. Wipes saturated with water were ineffective against either *B. subtilis* spores or *P. aeruginosa* cells.

### Experiment 3

### EVALUATION OF THE EFFECTIVENESS OF ClO₂ WIPES IN KILLING/ REMOVING METHICILLIN RESISTANT STAPHYLOCOCCUS AUREUS (MRSA) DRIED ONTO A STAINLESS STEEL TEST SURFACE

### Test Method

The following test method was used to evaluate the effectiveness of ClO₂ Wipes in killing/ removing test-organisms dried onto test surfaces. The test method involves the following steps:
1. Mark out an 18 inch (457.2 mm) square on the test surface.
2. Inoculate test surface with 4.5 ml of test organism suspension.
3. Spread inoculum over 18 inch (457.2 mm) square test area using a plastic spreader.
4. Allow inoculum to dry (30-60 minutes).
5. Don pair of disposable plastic gloves.
6. Prepare a ClO₂ Wipe using the prescribed scrunch time (15 seconds).
7. Wipe test area for the prescribed wipe time (30 seconds).
8. Place the ClO₂ Wipe in 20 ml of universal neutraliser in a universal bottle. Vortex stir to release organisms. (Test Suspension A).
9. Swab entire test area with a cotton-tipped swab. Dip swab into 10 ml of universal neutraliser in a universal bottle and rotate cotton-tip against the inner wall of the bottle to release organisms. Repeat 10 times. Finally, snap off cotton-tip into the neutraliser. Vortex stir to release organisms. (Test Suspension B).
10. Prepare 5 serial deci-dilutions of Test Suspension A and Test Suspension B in diluent.
11. Inoculate 0.5 ml of each dilution onto a culture plate and spread using a plastic spreader. Incubate plates. Viable count.
12. Calculate log₁₀ reductions achieved from the difference in the initial inoculum and the number of test organisms recovered after cleaning/ disinfection with a ClO₂ Wipe.
13. Repeat above using control wipes (70% IPA, 1% Hospec & sterile water).

### Variables selected in Experiment 3

### Test surface

A flat stainless steel laboratory bench.

### Test organism

Methicillin Resistant Staphylococcus aureus (MRSA): a clinical isolate from the Royal Preston Hospital.

### Inoculum

The test surface was inoculated with >10⁹ bacterial cells: 4.5 ml of an overnight culture in Nutrient Broth.

### Suspending fluid

Nutrient Broth

### Scrunch time

15 seconds

### Wipe time

30 seconds

### Controls

1. 70% IPA wipe: Azowipe (Vernon Carus).
2. 1% Hospec general purpose neutral liquid detergent (Young's Detergents) / Kimcare Medical Wipe (Kimberly-Clark). The wipe was immersed in 1% Hospec and then squeezed with the fingers to remove excess fluid.
3. Sterile deionised water / Kimcare Medical Wipe (Kimberly-Clark). The wipe was immersed in water and then squeezed with the fingers to remove excess fluid.

### Results

**Table 4**

| Exp | Disinfectant/detergent | Mixing time (sec) | Wiping time (sec) | Viable Count | |
|---|---|---|---|---|---|
| | | | | Surface | Wipe |
| 1 | ClO₂ | 15 | 30 | 0 | 0 |
| 2 | ClO₂ (repeat) | 15 | 30 | 0 | 0 |
| 3 | 70% IPA | | 30 | 5.5 x 10⁴ | 9 |
| 4 | 1% Hospec | | 30 | 5.5 x 10⁴ | 6.0 x 10⁴ |
| 5 | Deionised H₂O | | 30 | 5.7 x 10⁴ | 5.9 x 10⁴ |

**Table 5**

| Exp | Disinfectant/detergent | Mixing time (sec) | Wiping time (sec) | Total number of organisms recovered | |
|---|---|---|---|---|---|
| | | | | Surface¹ | Wipe² |
| 1 | ClO₂ | 15 | 30 | 0 | 0 |
| 2 | ClO₂ (repeat) | 15 | 30 | 0 | 0 |
| 3 | 70% IPA | | 30 | 1.1 x 10⁶ | 3.6 x 10² |
| 4 | 1% Hospec | | 30 | 1.1 x 10⁶ | 2.4 x 10⁶ |
| 5 | Deionised H₂O | | 30 | 1.1 x 10⁶ | 2.4 x 10⁶ |

| | | | | | |
|---|---|---|---|---|---|
| ¹ Viable Count in Table 1 x 20 (0.5 ml of 10 ml neutraliser plated out). ² Viable Count in Table 1 x 40 (0.5 ml of 20 ml neutraliser plated out). | | | | | |

### Interpretation of results

1. Wiping with a ClO₂ Wipe for 30 seconds was completely effective. No test organisms were recovered from either the test surface or the wipes in duplicate experiments.
2. Wiping the test surface with a 70% IPA wipe (Azowipe) for 30 seconds was ineffective. This could be due to:
   (a) an exposure time of 30 seconds was not long enough to kill the MRSA
   (b) the IPA evaporated off the test surface before the minimum exposure time required to kill the MRSA
   (c) the volume of IPA on the wipe was insufficient to deal with the >10⁹ MRSA dried onto the 18 inch test surface
   (d) a combination of the above.
3. Only 360 test organisms were recovered from the Azowipe. This could be due to :
   (a) entrapment of test organisms in the fibres
   (b) incomplete/ slow neutralisation of the residual IPA on the wipe by the neutraliser
   (c) a combination of the above
4. Wipes saturated with either 1% Hospec or sterile water were ineffective.

### Experiment 4

This experiment was carried out to evaluate the effectiveness of ClO₂ Wipes in killing/ removing spores of *Bacillus subtilis var. niger* NCTC 10073 dried out for 24 h at room temperature on a stainless steel test surface.

### Test Method

1. Mark out a 12 inch (304.8 mm) square on the test surface.
2. Inoculate test surface with 1.0 ml of aqueous spore suspension.
3. Spread inoculum over 12 inch (304.8 mm) square test area using a plastic spreader.
4. Allow inoculum to dry out naturally at room temperature for 24 h.
5. Don pair of disposable plastic gloves.
6. Prepare a ClO₂ Wipe using the prescribed scrunch time (15 seconds).
7. Wipe test area for the prescribed wipe time (30 seconds).
8. Place the ClO₂ Wipe in 20 ml of universal neutraliser in a universal bottle. Vortex stir to release organisms. (Test Suspension A).
9. Swab entire test area with a cotton-tipped swab. Dip swab into 10 ml of universal neutraliser in a universal bottle and rotate cotton-tip against the inner wall of the bottle to release organisms. Repeat 10 times. Finally, snap off cotton-tip into the neutraliser. Vortex stir to release organisms. (Test Suspension B).
10. Prepare 5 serial deci-dilutions of Test Suspension A and Test Suspension B in diluent.
11. Inoculate 0.5 ml of each dilution onto a culture plate and spread using a plastic spreader.
12. Repeat above using a control wipe (a Medical Wipe saturated with sterile water).
13. Incubate plates. Viable count.
14. Calculate log₁₀ reductions achieved using the ClO₂ Wipe from the difference in viable count obtained using the ClO₂ Wipe and the control wipe.

### Variables selected in Experiment 4

### Test surface

A flat stainless steel instrument tray.

### Test organism

*Bacillus subtilis var. niger* NCTC 10073. A spore suspension was prepared by the method of Beeby & Whitehouse.

### Inoculum

The test surface was inoculated with (a) 10⁶ spores, and (b) 10⁸ spores.

### Suspending fluid

Deionised water.

### Drying time

The inoculated instrument tray was allowed to dry out naturally at room temperature for 24 h in a dark cupboard.

### Scrunch time

15 seconds.

### Wipe time

30 seconds.

### Control

1. Sterile deionised water / Kimcare Medical Wipe (Kimberly-Clark). The wipe was immersed in water and then squeezed with the fingers to remove excess fluid.

### Results

**Table 6**

| Exp. | Inoculum (spores) | Disinfectant/detergent | Mixing time (sec) | Wiping time (sec) | Viable Count | |
|---|---|---|---|---|---|---|
| | | | | | Surface | Wipe |
| 1 | 10⁶ | ClO₂ | 15 | 30 | 0 | 0 |
| 2 | 10⁶ | Water | 15 | 30 | 2.0 x 10² | 2.1 x 10² |
| 3 | 10⁸ | ClO₂ | 15 | 30 | 4.8 x 10² | 1.3 x 10² |
| 4 | 10⁸ | Water | 15 | 30 | 6.6 x 10⁴ | 1.9 x 10⁵ |

**Table 7**

| Exp. | Inoculum (spores) | Disinfectant/detergent | Mixing time (sec) | Wipe time (sec) | Total number of spores recovered | |
|---|---|---|---|---|---|---|
| | | | | | Surface¹ | Wipe² |
| 1 | 10⁶ | ClO₂ | 15 | 30 | 0 | 0 |
| 2 | 10⁶ | Water | 15 | 30 | 4.0 x 10³ | 8.4 x 10³ |
| 3 | 10⁸ | ClO₂ | 15 | 30 | 9.6 x 10³ | 5.2 x 10³ |
| 4 | 10⁸ | Water | 15 | 30 | 1.3 x 10⁶ | 7.6 x 10⁶ |

| | | | | | | |
|---|---|---|---|---|---|---|
| ¹ Viable Count in Table 1 x 20 (0.5 ml of 10 ml neutraliser plated out). ² Viable Count in Table 1 x 40 (0.5 ml of 20 ml neutraliser plated out). | | | | | | |

### Interpretation of results

1. Spores dried out for 24 h at room temperature on a stainless steel test surface were not easy to dislodge using a Medical Wipe saturated with deionised water. With the 10⁶ inoculum the recovery was 4.0-8.4 x 10³ spores leaving 2-3 log₁₀ spores on the surface (assuming no mortality). With the 10⁸ inoculum the recovery was 1.3 -7.6 x 10⁶ spores leaving 1-2 log₁₀ spores on the surface.
2. ClO₂ Wipes were effective in killing/ removing spores dried out for 24 h at room temperature on the stainless steel test surface. With the 10⁶ inoculum, no spores were recovered from either the surface or wipe which represents a 3-4 log₁₀ reduction on both the surface and wipe. With the 10⁸ inoculum, a 2-3 log₁₀ reduction of spores was achieved on the surface and a 3-4 log₁₀ reduction on the wipe.

Thus, the invention provides a sterilant system which can be prepared *in situ* and which provides bactericidal, fungicidal, virucidal, and sporicidal fabrics. The system is particularly useful for sterilising wipes and for the dressing of wounds and ulcers.

It is appreciated that certain features of the invention which are, for clarity, described in the context of separate embodiments, may also be provided in combination in a single embodiment. Conversely, various features of the invention which are, for brevity, described in the context of a single embodiment, may also be provided separately, or in any suitable combination. It is to be recognized that various alterations, modifications, and/or additions may be introduced into the constructions and arrangements of parts described above without departing from the ambit of the present invention. As used herein, the indefinite articles `a' and 'an' connote 'one or more' unless the context requires otherwise.

## Claims

1. A two-part sterilant system comprising:
(a) a first part comprising a first reagent in a carrier medium; and
(b) a second part which is miscible with the first part and which comprises a second reagent in a carrier medium;
wherein the first reagent and the second reagent will react when mixed to provide a sterilising composition;
**characterised in that** the first part is contained in a pump dispenser and either:
will be dispensed as a gel; or
is a fluid which includes a foam promoter whereby it will be dispensed as a foam;
and **in that** the second part is absorbed or impregnated in at least one fabric member in a sealed container.

2. A sterilant system according to claim 1, wherein the pump dispenser is a trigger-operated dispenser.

3. A sterilant system according to claim 1 or claim 2, wherein the at least one fabric member comprises a plurality of fabric wipes, each of which is provided in its own separate sealed sachet.

4. A sterilant system according to any preceding claim, wherein at least one of the first part and the second part includes an indicator reagent that changes colour when the parts are mixed together.

5. A sterilant system according to claim 4, wherein the first part and the second part have a different pH and wherein the indicator reagent changes colour in response to a change in pH when the parts are mixed.

6. A sterilant system according to any preceding claim, wherein one of the first part and the second part comprises a solution containing sodium chlorite or sodium chlorate and the other comprises an acidic solution.

7. A sterilant system according to claim 6, wherein the acidic solution comprises a solution of citric acid, sorbic acid and boric acid.

8. A sterilant system according to any preceding claim, wherein said first part is a fluid which includes a foam promoter and wherein said foam promoter is present in an amount from 0.1 to 50% w/w of said first part.

9. A sterilant system according to claim 8, wherein said foam promoter is present in an amount from 3 to 6% w/w of said first part.

10. A sterilant system according to any preceding claim, wherein one of the first part and the second part further includes a humectant, said humectant being present in an amount from 0.1 to 50% w/w of said first part or said second part.

11. A sterilant system according to claim 10, wherein said humectant is present in an amount from 1 to 3% w/w of said first part or said second part.

12. A sterilant system according to any preceding claim, wherein when equal weights of the first part and the second part are mixed they provide a sterilising composition having a pH of from 4.5 to 6.5.

13. A sterilant system according to claim 6, wherein the first part comprises said solution of sodium chlorite or sodium chlorate.

14. A sterilant system according to claim 13, wherein said first part comprises a solution of sodium chlorite and said acidic solution comprises an aqueous mixture of citric acid, sorbic acid, boric acid, and a buffer.

15. A sterilant system according to claim 1, wherein one of the first reagent and the second reagent comprises chlorite and the other comprises hypochlorite.

16. A sterilant system according to claim 1, wherein one of the first reagent and the second reagent comprises chlorate and the other comprises a peroxide and an acid.

17. A two-part wound-dressing system comprising:
(a) a first part comprising a first reagent in a carrier medium; and
(b) a second part which is miscible with the first part and which comprises a second reagent in a carrier medium;
wherein the first reagent and the second reagent will react when mixed to provide a sterilising composition; **characterised in that** the first part is contained in a pump dispenser and either:
will be dispensed as a gel; or
is a fluid which includes a foam promoter whereby it will be dispensed as a foam;
and **in that** the second part is absorbed or impregnated in at least one fabric member in a sealed container.

18. A wound-dressing system according to claim 17, wherein the second part is absorbed or impregnated in a plurality of fabric wound dressings each of which is provided in its own separate sealed sachet.

## Patentansprüche

1. Zweiteiliges Sterilisiersystem umfassend
(a) einen ersten Teil umfassend ein erstes Reagenz in einem Trägermedium und
(b) einen zweiten Teil, der mit dem ersten Teil mischbar ist und der ein zweites Reagenz in einem Trägermedium umfasst,
wobei das erste Reagenz und das zweite Reagenz beim Mischen zur Bereitstellung einer Sterilisierzusammensetzung reagieren,
**dadurch gekennzeichnet, dass**
der erste Teil in einem Pumpspender enthalten ist
und entweder als Gel abgegeben wird
oder ein ein Mittel zur Förderung der Schaumbildung enthaltendes Fluid ist, wodurch es als Schaum abgegeben wird
und **dadurch, dass** der zweite Teil in mindestens einem Gewebeteil in einem versiegelten Behälter absorbiert oder imprägniert ist.

2. Sterilisiersystem nach Anspruch 1, worin der Pumpspender ein mit einem Auslöser betriebener Spender ist.

3. Sterilisiersystem nach Anspruch 1 oder 2, worin das mindestens eine Gewebeteil eine Vielzahl von Gewebewischtüchern umfasst, von denen jedes in seinem eigenen versiegelten Päckchen bereitgestellt wird.

4. Sterilisiersystem nach einem der vorangehenden Ansprüche, worin mindestens einer des ersten und des zweiten Teils ein Indikatorreagenz enthält, das die Farbe wechselt, wenn die Teile miteinander gemischt werden.

5. Sterilisiersystem nach Anspruch 4, worin der erste und der zweite Teil einen unterschiedlichen pH-Wert haben und worin das Indikatorreagenz als Reaktion auf eine Änderung des pH-Werts die Farbe wechselt, wenn die Teile gemischt werden.

6. Sterilisiersystem nach einem der vorangehenden Ansprüche, worin einer des ersten und zweiten Teils eine Natriumchlorit oder Natriumchlorat enthaltende Lösung und der andere eine Säurelösung umfasst.

7. Sterilisiersystem nach Anspruch 6, worin die Säurelösung eine Lösung von Zitronensäure, Sorbinsäure und Borsäure umfasst.

8. Sterilisiersystem nach einem der vorangehenden Ansprüche, worin der erste Teil ein ein Mittel zur Förderung der Schaumbildung enthaltendes Fluid ist und worin das Mittel zur Förderung der Schaumbildung in einer Menge von 0,1 bis 50 Gew.-% des ersten Teils vorliegt.

9. Sterilisiersystem nach Anspruch 8, worin das Mittel zur Förderung der Schaumbildung in einer Menge von 3 bis 6 Gew.-% des ersten Teils vorliegt.

10. Sterilisiersystem nach einem der vorangehenden Ansprüche, worin einer des ersten Teils und des zweiten Teils ein Befeuchtungsmittel beinhaltet, worin das Befeuchtungsmittel in einer Menge von 0,1 bis 50 Gew.-% des ersten Teils oder des zweiten Teils vorliegt.

11. Sterilisiersystem nach Anspruch 10, worin das Befeuchtungsmittel in einer Menge von 1 bis 3% Gew.-% des ersten oder des zweiten Teils vorliegt.

12. Sterilisiersystem nach einem der vorangehenden Ansprüche, worin, wenn Äquivalentgewichte des ersten und des zweiten Teils gemischt werden, diese eine Sterilisierzusammensetzung mit einem pH-Wert von 4,5 bis 6,5 bereitstellen.

13. Sterilisiersystem nach Anspruch 6, worin der erste Teil die Lösung von Natriumchlorit oder Natriumchlorat umfasst.

14. Sterilisiersystem nach Anspruch 13, worin der erste Teil eine Lösung von Natriumchlorit und die Säurelösung eine wässrige Mischung von Zitronensäure, Sorbinsäure, Borsäure und einem Puffer umfasst.

15. Sterilisiersystem nach Anspruch 1, worin eines des ersten und des zweiten Reagenzes Chlorit und das andere Hypochlorit umfasst.

16. Sterilisiersystem nach Anspruch 1, worin eines des ersten und des zweiten Reagenzes Chlorat und das andere Peroxid und eine Säure umfasst.

17. Aus zwei Teilen bestehendes Wundverbandsystem, umfassend
(a) einen ein erstes Reagenz in einem Trägermedium umfassenden ersten Teil und
(b) einen mit dem ersten Teil mischbaren zweiten Teil, der ein zweites Reagenz in einem Trägermedium umfasst,
worin das erste Reagenz und das zweite Reagenz beim Mischen zur Bereitstellung einer Sterilisierzusammensetzung reagieren,
**dadurch gekennzeichnet, dass**
der erste Teil in einem Pumpspender enthalten ist
und entweder als Gel abgegeben wird
oder ein ein Mittel zur Förderung der Schaumbildung beinhaltendes Fluid ist, wodurch es als Schaum abgegeben wird,
und **dadurch, dass** der zweite Teil in mindestens einem Gewebeteil in einem versiegelten Behälter absorbiert oder imprägniert ist.

18. Wundverbandsystem nach Anspruch 17, worin der zweite Teil in einer Vielzahl von Wundverbänden aus Gewebe absorbiert oder imprägniert ist, von denen jeder in seinem eigenen versiegelten Päckchen bereitgestellt wird.

## Revendications

1. Système stérilisant en deux parties :
(a) une première partie comprenant un premier réactif dans un milieu de support ; et
(b) une seconde partie qui est miscible avec la première partie et qui comprend un second réactif dans un milieu de support ;
dans lequel le premier réactif et le second réactif réagiront lorsqu'ils seront mélangés de façon à donner une composition de stérilisation ;
**caractérisé en ce que** la première partie est contenue dans un flacon à bouchon gicleur et :
sera distribuée sous la forme d'un gel ; ou
est un fluide qui comprend un agent moussant grâce auquel il sera distribué sous la forme d'une mousse ;
et **en ce que** la seconde partie est absorbée ou imprégnée dans au moins un élément en tissu dans un récipient scellé.

2. Système stérilisant selon la revendication 1, dans lequel le flacon à bouchon gicleur est un flacon fonctionnant avec une gâchette.

3. Système stérilisant selon la revendication 1 ou la revendication 2, dans lequel le ou les éléments en tissu comprennent une pluralité de lingettes en tissu, chacune étant disposée dans son propre sachet scellé séparé.

4. Système stérilisant selon l'une quelconque des revendications précédentes, dans lequel au moins une parmi la première partie et la seconde partie comprend un réactif indicateur qui change de couleur lorsque les parties sont mélangées ensemble.

5. Système stérilisant selon la revendication 4, dans lequel la première partie et la seconde partie ont un pH différent et dans lequel le réactif indicateur change de couleur en réponse à un changement du pH lorsque les parties sont mélangées.

6. Système stérilisant selon l'une quelconque des revendications précédentes, dans lequel une parmi la première partie et la seconde partie comprend une solution contenant du chlorite de sodium ou du chlorate de sodium et l'autre comprend une solution acide.

7. Système stérilisant selon la revendication 6, dans lequel la solution acide comprend une solution d'acide citrique, d'acide sorbique et d'acide borique.

8. Système stérilisant selon l'une quelconque des revendications précédentes, dans lequel ladite première partie est un fluide qui comprend un agent moussant et dans lequel ledit agent moussant est présent en une quantité allant de 0,1 à 50 % en poids de ladite première partie.

9. Système stérilisant selon la revendication 8, dans lequel ledit agent moussant est présent en une quantité allant de 3 à 6 % en poids de ladite première partie.

10. Système stérilisant selon l'une quelconque des revendications précédentes, dans lequel une parmi la première partie et la seconde partie comprend en outre un agent mouillant, ledit agent mouillant étant présent en une quantité allant de 0,1 à 50 % en poids de ladite première partie ou de ladite seconde partie.

11. Système stérilisant selon la revendication 10, dans lequel ledit agent mouillant est présent en une quantité allant de 1 à 3 % en poids de ladite première partie ou de ladite seconde partie.

12. Système stérilisant selon l'une quelconque des revendications précédentes, dans lequel lorsque des masses identiques de la première partie et de la seconde partie sont mélangées, on obtient une composition de stérilisation ayant un pH allant de 4,5 à 6,5.

13. Système stérilisant selon la revendication 6, dans lequel la première partie comprend ladite solution de chlorite de sodium ou de chlorate de sodium.

14. Système stérilisant selon la revendication 13, dans lequel la première partie comprend une solution de chlorite de sodium et ladite solution acide comprend un mélange aqueux d'acide citrique, d'acide sorbique, d'acide borique et un tampon.

15. Système stérilisant selon la revendication 1, dans lequel un parmi le premier réactif et le second réactif comprend un chlorite et l'autre comprend un hypochlorite.

16. Système stérilisant selon la revendication 1, dans lequel un parmi le premier réactif et le second réactif comprend un chlorate et l'autre comprend un peroxyde et un acide.

17. Système de pansement en deux parties comprenant :
(a) une première partie comprenant un premier réactif dans un milieu de support ; et
(b) une seconde partie qui est miscible avec la première partie et qui comprend un second réactif dans un milieu de support ;
dans lequel le premier réactif et le second réactif réagiront lorsqu'ils seront mélangés de façon à donner une composition de stérilisation ;
**caractérisé en ce que** la première partie est contenue dans un flacon à bouchon gicleur et :
sera distribuée sous la forme d'un gel ; ou
est un fluide qui comprend un agent moussant grâce auquel il sera distribué sous la forme d'une mousse ;
et **en ce que** la seconde partie est absorbée ou imprégnée dans au moins un élément en tissu dans un récipient scellé.

18. Système de pansement selon la revendication 17, dans lequel la seconde partie est absorbée ou imprégnée dans une pluralité de pansements en tissu, chacun étant disposé dans son propre sachet scellé séparé.
